# EUROPÄISCHE PATENTSCHRIFT

(11) **EP 1 962 734 B1**
(45) Veröffentlichungstag und Bekanntmachung des Hinweises auf die Patenterteilung: **10.06.2009**
(21) Anmeldenummer: 06828725.9
(22) Anmeldetag: 21.12.2006
(51) Int. Cl.: A61F 2/58, F16F 1/54

(54) **HANDPROTHESE**
HAND PROSTHESIS
PROTHESE DE MAIN

(30) Priorität: 22.12.2005 DE 102005062083
(43) Veröffentlichungstag der Anmeldung: 03.09.2008
(73) Patentinhaber: Otto Bock HealthCare GmbH, 37115 Duderstadt (DE)
(72) Erfinder: PUCHHAMMER, Gregor, A-1130 Wien (AT)
(74) Vertreter: Stornebel, Kai
(86) Internationale Anmeldenummer: PCT/DE2006/002310
(87) Internationale Veröffentlichungsnummer: WO 2007/076833

(56) Entgegenhaltungen:
- WO-A-03/094807
- DE-C- 292 785
- DE-C- 307 250
- DE-C- 307 338
- FR-A- 1 112 526
- GB-A- 1 517 035
- US-A- 5 549 712
- US-A1- 2004 015 240

## Beschreibung

Die Erfindung betrifft eine Handprothese mit einem Chassis und Kopplungselementen zur Befestigung der Handprothese an einem Armstumpf sowie mit zumindest einem Gelenk zur Flexion und Extension der Handprothese relativ zu den Kopplungselementen, wobei das Chassis entgegen einer Federkraft in einer Neutralstellung gehalten ist.

Aus der US 2004/0015240 A1 ist eine Handprothese mit Greifelementen an einem Chassis bekannt, das ein halbkreisförmiges Drehelement aufweist. Eine Drehfeder ist zwischen einer Basisplatte und einer Montageplatte angeordnet und hält das Chassis mit den motorisch angetriebenen Greifelementen in einer Neutralstellung. Über eine verschiebbare Verriegelungsplatte kann das Chassis in unterschiedlichen Winkelstellungen verriegelt werden.

Die WO 03/094807 A1 betrifft eine prothetische Gliedmaße zur Verwendung als ein Teil eines Unterschenkels. Ein Prothesenfuß ist über eine Hülse mit elastisch deformierbaren Elementen schwenkbar an einem Unterschenkelschaft gelagert. Bei einem Verschwenken des Fußes relativ zu dem Unterschenkelschaft erhöhen sich die Rückstellkräfte im Umfang der Drehung der Elemente zueinander.

Aufgabe der vorliegenden Erfindung ist es, eine Handprothese dahingehend zu verbessern, dass sie ein möglichst natürliches Erscheinungsbild bereitstellt.

Erfindungsgemäß wird diese Aufgabe durch eine Handprothese mit den Merkmalen des Anspruchs 1 gelöst. Vorteilhafte Ausgestaltungen und Weiterbildungen der Erfindung sind in den Unteransprüchen aufgeführt.

Die erfindungsgemäße Handprothese mit einem Chassis und Kopplungselementen zur Befestigung der Handprothese an einem Armstumpf sowie mit zumindest einem Gelenk zur Flexion oder Extension der Handprothese relativ zu den Kopplungselementen, wobei das Chassis entgegen einer Federkraft in einer Neutralstellung gehalten ist, sieht zumindest ein Federelement vor, das dem Chassis zugeordnet ist, das eine progressive Federkennlinie aufweist und mit zunehmenden Flexions- oder Extensionswinkeln eine zunehmende Gegenkraft bereitstellt. Zur Erzielung eines möglichst natürlichen Erscheinungsbildes der Handprothese ist vorgesehen, eine natürliche Hand in ihrem Erscheinungsbild sowohl in Funktion als auch in Aussehen möglichst naturgetreu nachzuahmen, um eine verbesserte Akzeptanz bei den Prothesenträgern bereitzustellen. Damit bei Berührung von Mitmenschen oder Gegenständen nicht der Eindruck eines künstlichen Körperteiles entsteht, ist ein weiches Ausformen aller an der Oberfläche liegenden Prothesenteile und das gezielte Einbauen von elastischen Teilen in die Hand und insbesondere in den Handgelenksbereich notwendig. Dabei ist das Gelenk der Handprothese als solches als ein loses Gelenk ausgebildet, das durch zumindest ein Federelement in einer Neutral- oder Ausgangsstellung gehalten wird. Dieses Federelement ist dem Chassis zugeordnet und stützt dieses gegenüber dem anderen Gelenksteil, insbesondere den Kopplungselementen oder zwischengeordneten Komponenten ab. Das Federelement ist dabei so ausgebildet, dass eine progressive Federkennlinie bei zunehmenden Flexions- oder Extensionswinkeln vorhanden ist, so dass in der Neutralstellung der Hand eine weiche Charakteristik und eine leichte Beweglichkeit innerhalb der Neutralstellung und in einem geringen Winkelbereich darum möglich ist. Gegen die jeweiligen Endstellungen des Handchassis wird eine erhöhte Gegenkraft bereitgestellt, um ein hartes Anschlagen an den Bewegungsgrenzen zu vermeiden. Dies erhöht den natürlichen Charakter der Handprothese.

Das Federelement kann als eine Torsionsfeder ausgebildet sein, die sowohl in Extensionsrichtung als auch in Flexionsrichtung wirksam ist. Alternativ kann je ein Federelement entgegen der Extension und Flexion wirksam sein, wobei auch mehrere Federkomponenten, die nacheinander wirksam werden, ein Federelement ausbilden können. Ebenfalls ist es möglich, dass das Federelement als ein Elastomerteil ausgebildet ist, das in Abhängigkeit von dem Flexions- oder Extensionswinkel eine erhöhte, insbesondere progressive Federkraft der Verdrehung um das Flexionsgelenk entgegensetzt. Das oder die Federelemente können auswechselbar ausgebildet sein, um eine Anpassung an die jeweilige Handprothese oder Bedürfnisse der Prothesennutzer zu ermöglichen.

Neben einer alleinigen Flexion und Extension ist es vorgesehen, dass das Chassis um eine Achse im Wesentlichen parallel zu der Längserstreckung des Armstumpfes drehbar gelagert ist, um eine weitere Bewegungskomponente in dem Handgelenk für ein möglichst natürliches Erscheinungsbild der Handprothese bereitstellen zu können.

Um nach dem Greifen eines Gegenstandes diesen anheben und halten zu können, ist vorgesehen, dass eine Sperrvorrichtung in dem Gelenk vorhanden ist, die das Gelenk in einer gewählten Stellung arretiert. Diese Sperrvorrichtung ist sowohl für das Flexions-Extensions-Gelenk als auch für das rotatorische Gelenk vorgesehen. Grundsätzlich kann eine solche Sperrvorrichtung auch separat in eine Handprothese eingebaut werden, ohne dass eine entsprechende Federcharakteristik oder Rückstellvorrichtung vorhanden sein muss. Die Sperrvorrichtung kann dabei insbesondere eine Planverzahnung aufweisen, die zur Arretierung des Gelenkes in Eingriff bringbar ist. Dies hat den Vorteil einer leichten Lösbarkeit und einer sehr feinen Winkelteilung, so dass die Handprothese in nahezu jeder beliebigen Winkelstellung arretierbar ist.

Um eine möglichst natürliche Anmutung der Handprothese bereitzustellen zu können, ist das Flexions-Extensions-Gelenk im Mittelhandbereich des Chassis angeordnet. Der Mechanismus für die Sperrfunktion kann, wie bei einem Kugelschreiber, durch einmaliges Betätigen eingeschaltet und durch ein wiederholtes Betätigen ausgeschaltet werden. Die Betätigung der Sperrvorrichtung kann entweder manuell über einen Druckknopf oder über eine elektrische betätigbare Antriebseinheit betätigt werden.

Die Ausrichtung der Gelenkachse des Extensions-Flexions-Gelenks in einem schiefen, insbesondere stumpfen Winkel zu der Längserstreckung des Armstumpfes kombiniert die Eigenschaften eines Flektierens und einer gleichzeitigen Abduktion der Handprothese. Dies ist für einen Großteil der täglichen Verrichtungen günstig.

Nachfolgend wird ein Ausführungsbeispiel der Erfindung anhand der beigefügten Figuren näher erläutert. Es zeigen:
- Figur 1 -: eine Handprothese in Gesamtdarstellung in Draufsicht;
- Figur 2 -: eine Handprothese gemäß Figur 1 in Seitenansicht;
- Figur 3 -: eine perspektivische Explosionszeichnung eines Handprothesengelenkes;
- Figur 4:: eine Schnittdarstellung des Prothesengelenkes in Neutralstellung;
- Figur 5 -: einen Schnitt gemäß A-A der Figur 4;
- Figur 6 -: eine Darstellung gemäß Figur 4 in einer um 60° verdrehten Stel- lung;
- Figur 7 -: eine perspektivische, geschnittene Explosionsdarstellung einer Flexionssperre; sowie
- Figur 8 -: eine Schnittdarstellung der Flexionssperre gemäß Figur 7 in mon- tierter Stellung.

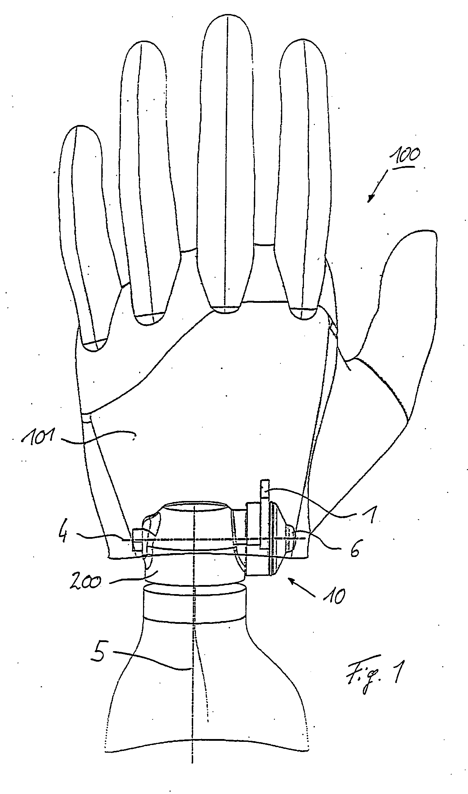

In der Figur 1 ist in Draufsicht in Gesamtdarstellung eine Handprothese 100 mit einem Chassis 101 gezeigt, das über eine Gelenkeinrichtung 10 um eine Schwenkachse 4 sowohl in Flexionsrichtung als auch Extensionsrichtung schwenkbar gelagert ist. Die Gelenkeinrichtung 10, nachfolgend Gelenk genannt, weist eine Montageplatte 1 zur Befestigung an dem Handchassis 101 auf. Weiterhin ist in dem Gelenk 10 ein Gehäuse 200 zur Kopplung der Handprothese 100 mit Befestigungseinrichtungen an einem Armstumpf ausgebildet. Das Gehäuse 200 kann über ein Kopplungselement 2 mit der Montageplatte 1 verbunden sein. Ebenfalls ist es vorgesehen, dass das Gehäuse 200 und damit de Handprothese 100 eine Drehbewegung um eine Achse 5 ermöglicht. Die Drehachse 5 ist im Wesentlichen parallel zur Längserstreckung des Unterarmschaftes ausgerichtet. Die Flexions- und Extensionsschwenkachse 4 steht in einem rechten Winkel zu der Drehachse 5.

An dem Gelenk 10 ist seitlich eine elastische Betätigungseinrichtung 6 für eine innerhalb des Gelenks 10 angeordnete Sperrvorrichtung angeordnet. Über das Betätigungselement 6 kann das Handchassis 101 in einer gewählten Winkelstellung verriegelt werden. Ebenfalls kann das Handchassis 101 aus der verriegelten Stellung in eine entriegelte Stellung durch erneutes Betätigen überführt werden.

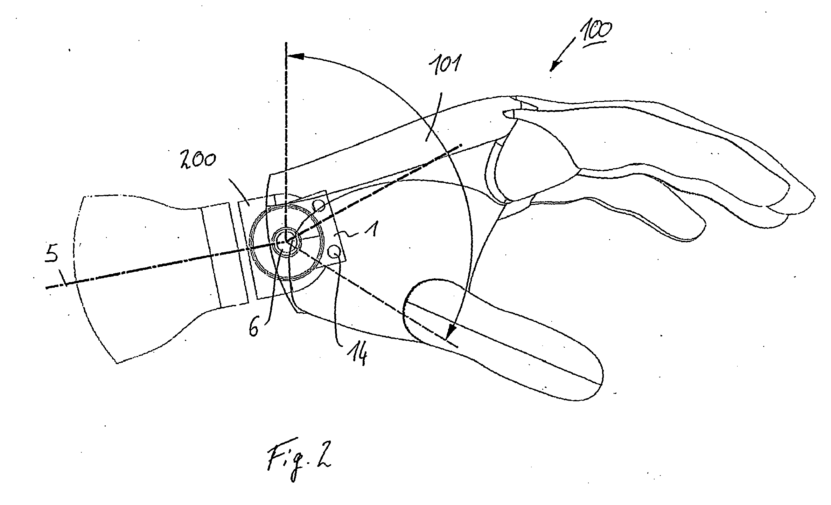

In der Figur 2 ist in Seitenansicht die Handprothese 100 mit dem Chassis 101 sowie der Montageplatte 1 gezeigt, an der das Handchassis 101 über Bohrungen 14 und geeignete Befestigungsmittel festgelegt ist. Die Handprothese 100 befindet sich in der Figur 2 in der Neutralstellung und kann eine Extensionsbewegung entgegen dem Uhrzeigersinn sowie eine Flexionsbewegung im Uhrzeigersinn ausführen, wie durch den Doppelpfeil angedeutet ist. Die Finger können durch einen Motor innerhalb des Chassis 101 oder des Gehäuses 200 einzeln oder gemeinsam angetrieben werden. Die Handprothese 100 wird über ein Federelement in dem Gelenk 10 in der Neutralstellung gehalten, wodurch eine leichte Beweglichkeit des Handchassis 101 in die Neutralstellung möglich ist.

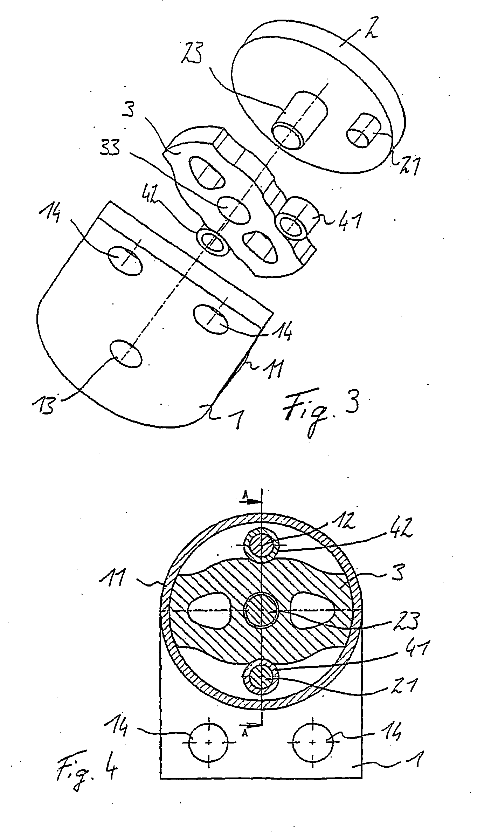

In der Figur 3 ist die Montageplatte 1 der in dieser Figur nicht weiter dargestellten Handprothese mit einem kreisförmigen Rahmen 11 gezeigt. In der Montageplatte 1 sind Bohrungen 14 zur Befestigung weiterer Komponenten der Handprothese ausgebildet. Ebenfalls ist eine zentrale Bohrung 13 im Mittelpunkt des Rahmens 11 zur Aufnahme eines Drehzapfens 23 eines Kopplungselementes 2 ausgebildet. Zwischen dem Kopplungselement 2 und der Montageplatte 1 oder einem entsprechenden Element des Chassis 101 ist ein Federelement 3 in Gestalt eines Elastomerkörpers angeordnet. Das Federelement 3 weist eine zentrale Bohrung 33 auf, in der der Drehzapfen 23 in montierter Stellung eingeführt ist. Der Drehzapfen 23 ragt in die Bohrung 13 der Monatgeplatte 1 in montierter Stellung hinein.

An dem Kopplungselement 2, das seinerseits an einem Befestigungsteil oder dem Gehäuse 200 zur Aufnahme an einem Armstumpf festgelegt sein kann, ist ein Stift 21 angeordnet oder ausgebildet, der eine Drehhülse 41 aufnimmt. An der Montageplatte 1 ist ebenfalls ein Stift 12, der in der Figur 3 nicht zu erkennen ist, ausgebildet, der eine gegenüberliegende Drehhülse 42 aufnimmt. Die Drehhülsen 41, 42 liegen auf der Oberfläche des Federelementes 3 auf und können darauf abrollen.

In der Figur 4 ist das Prothesenhandgelenk 10 in einer Schnittdarstellung in Neutralstellung gezeigt. Die beiden Drehhülsen 41, 42 sind auf ihren jeweiligen Lagerstiften 21, 12 angeordnet und liegen einander gegenüber an Ausbuchtungen 34 des Federelementes 3 an. Das Federelement 3 ist in dem kreisförmigen Rahmen 11, der an der Montageplatte 1 ausgebildet ist, eingelegt, wobei der äußere Umfang des Federelementes 3 abschnittsweise der Innenkontur des Rahmens 11 entspricht. Vorliegend ist das Federelement 3 spiegelsymmetrisch ausgebildet, so dass sowohl in Extensions- als auch in Flexionsrichtung gleiche Federraten vorliegen. Dies kann je nach Einsatzzweck variiert werden, so dass unterschiedliche Federkräfte bei Extension und Flexion vorliegen. In dem Federelement sind Ausnehmungen 35 ausgebildet, die das Federverhalten beeinflussen. Je größer die Ausnehmungen 35 sind, desto weicher ist die Lagerung der Handprothese um die Neutralstellung.

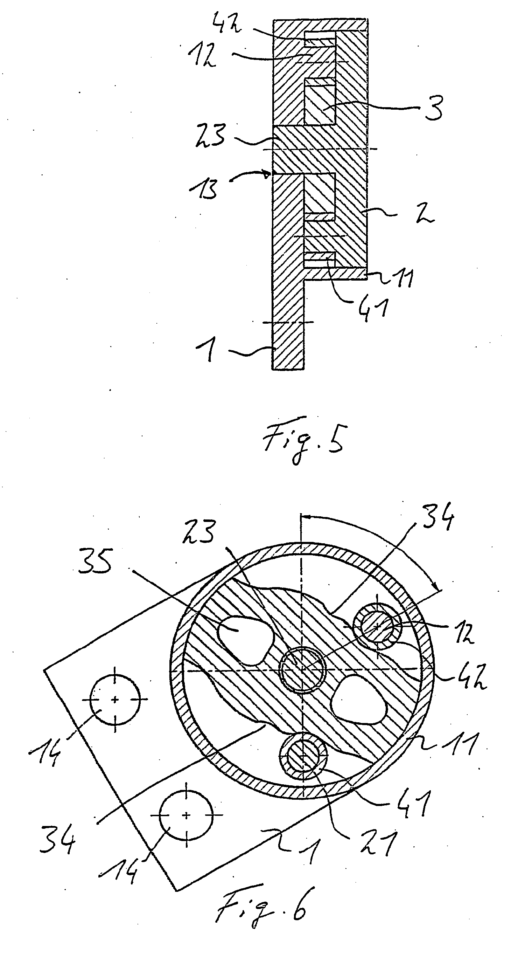

In der Figur 5 ist in einer Schnittdarstellung zu erkennen, dass der Rahmen 11 einstückig mit der Montageplatte 1 ausgebildet ist, ebenso wie der Lagerstift 12 für die Drehhülse 42. An dem Kopplungselement 2 ist der Lagerstift 21 einstückig ausgebildet, ebenso wie der Drehzapfen 23.

Wird nun die Handprothese 100 zusammen mit der Montageplatte 1 relativ zu dem Kopplungselement 2 verdreht, wie es in der Figur 6 dargestellt ist, verlagern sich die Lagerstifte 21, 12 relativ zueinander. Das Federelement 3, vorliegend als ein einteiliges Elastomerteil ausgebildet, wird zusammengedrückt, wobei durch die vorliegende Ausgestaltung des Federelementes 3 eine progressive Federcharakteristik erzielt werden kann. Dies kann beispielsweise durch eingefügte Komponenten innerhalb des Federelementes 3 und deren sequentielle Aktivierung in Abhängigkeit von dem Verdrehwinkel oder durch eine entsprechende Formgebung geschehen. In der Neutralstellung, wie sie in der Figur 3 dargestellt ist, weist das Federelement 3 eine relativ weiche Federcharakteristik auf, so dass die Handprothese 100 relativ locker, wie eine natürliche Hand, beim Bewegen des Unterarmes mitschwingt. Über die Einbuchtungen 34 wird eine Vorzugslage der Neutralstellung bewirkt, so dass die Handprothese 100 aufgrund der Rückstellkräfte des Federelementes 3 stets in diese zurückgestellt wird. Je weiter die Handprothese 100 relativ zu dem Kopplungselement 2 verdreht wird, erhöht sich die Gegenkraft durch das Federelement 3, wodurch ein hartes Anschlagen gegenüber Endanschlägen vermieden wird. Dies entspricht dem natürlichen Bewegungsablauf und Erscheinungsbild einer Hand und schont darüber hinaus die mechanischen Komponenten der Handprothese 100.

Alternativ zu der einteiligen Ausgestaltung des Federelementes 3 kann dieses auch aus mehreren separaten Federelementen ausgebildet sein. Beispielsweise können unterschiedliche Federelemente für die Extension und Flexion vorgesehen sein. Dann wird die Handprothese über zwei Federn in der Neutralstellung gehalten.

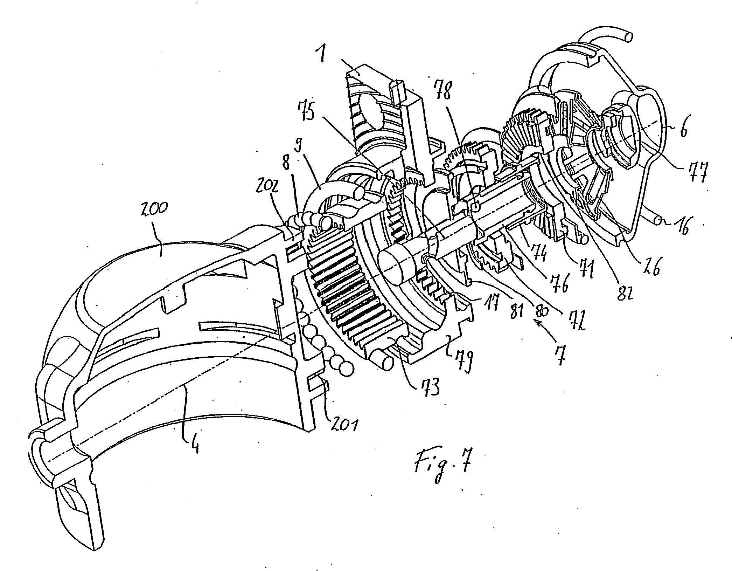

Die Figur 7 zeigt im Detail eine Sperrvorrichtung 7, die an dem Gehäuse 200 angebracht ist. Innerhalb der Sperrvorrichtung 7 ist die Montage- oder Basisplatte 1 zur Befestigung des Handchassis 101 angeordnet. In der dargestellten Ausführungsform der Sperrvorrichtung 7 ist das vorbeschriebene Federelement nicht gezeigt. Aufgrund der flachen Ausgestaltung des Federelementes 3 ist jedoch eine leichte Integration innerhalb der Sperrvorrichtung 7 oder bei dem Ansatz an das Gehäuse 200 möglich.

An dem Gehäuse 200 ist eine Außenverzahnung 201 mit einem radial umlaufenden Steg 202 ausgebildet. In dem Freiraum zwischen der Außenverzahnung 201 und dem Steg 202 wird ein Innenverzahnungsring 73 eingeführt, der sich zusammen mit dem Chassis 101 relativ zu der Basisplatte 1 verdrehen lässt. Die Drehung wird durch Lagerkugeln 8 erreicht, die in entsprechenden Kugelbahnen innerhalb des Innenverzahnungsringes 73 und dem Lagergehäuse 79 der Basisplatte 1 laufen. Ein Dichtring 9 dient als Abdichtung zwischen dem Gehäuse 200 und dem Lagergehäuse 79. Innerhalb des Lagergehäuses 79 ist im Bereich der Basisplatte 1 eine Innenverzahnung 17 ausgebildet. In den Innenverzahnungsring 73 greift eine Planverzahnungsscheibe 72 mit einer Außenverzahnung und einer Planverzahnung ein. Diese Planverzahnungsscheibe 72 steht im montierten Zustand bei verriegeltem Gelenk in Eingriff mit einer zweiten, gegenüberliegenden Planverzahnungsscheibe 71, die ebenfalls eine Außenverzahnung aufweist und in die Innenverzahnung des Lagergehäuses 79 eingreift. Die Planverzahnungsscheiben 71, 72 werden über einen Federring 80 und eine Tellerfeder 26 gegeneinander gedrückt und in Eingriff gehalten. Die Tellerfeder 26 stützt sich über eine Druckkappe 77 gegenüber einer elastischen Kappe 6 als Betätigungseinrichtung ab. Die elastische Kappe 6 wird über eine Haltering 16 an der Basisplatte 1 bzw. dem Lagergehäuse 79 wasserdicht festgelegt. Die Tellerfeder 26 und die Federscheibe 80 stützen sich über Druckscheiben 81, 82 an den Planverzahnungsscheiben 71, 72 ab.

Innerhalb der Planverzahnungsscheiben 71, 72 ist ein Ausrückmechanismus angeordnet, der bei Betätigung die Planverzahnungsscheiben 71, 72 in Axialrichtung auseinander bewegt. Die Druckkappe 77 kann in Richtung Gehäuse 200 verlagert werden und bewegt eine Druckhülse 76, in der ein Ausrückstift 75 und eine Kugel 78 geführt ist, in Richtung auf das Gehäuse 200. Die Tellerfedern 26 drücken die Druckscheibe 82 in Richtung auf das Gehäuse 200 und üben eine Kraft auf eine um die Druckhülse 76 angeordnete Ausrückhülse 74 aus. Die Ausrückhülse 74 hat einen umlaufenden Steg, der zwischen den Planverzahnungsscheiben 71, 72 hinein ragt und formschlüssig in Eingriff mit der dem Innenverzahnungsring 73 zugeordneten Planverzahnungsscheibe 72 treten kann. Die Kugel 78 ist innerhalb der Druckhülse 76 geführt und greift in den Ausrückstift 75 ein, in der eine Bahnführung, in Form der Abwicklung einer Herzkurve, wie bei einem Kugelschreiber, ausgebildet ist. Wird nun die Druckkappe in Richtung Gehäuse 200 bewegt, drückt die Tellerfeder 26 über die Druckscheibe 82 auf die Ausrückhülse 74 und bewegt die Planverzahnungsscheibe 72 axial von der Planverzahnungsscheibe 71 weg, so dass eine Rotation um die Gelenkachse 4 stattfinden kann. Bei weiterem Drücken der Druckkappe 77 beginnt die unter Vorspannung stehende Tellerfeder 26 einzuwandern, wobei die Kugel 78 auf dem feststehenden Ausrückstift 75, entlang der herzförmig ausgebildeten Bahnführung in Richtung feststehendes Gehäuse 200 geführt wird und bewirkt, dass die Druckhülse 76 an dem Ausrückstift 75 über die Kugel 78 festgehalten bleibt. Die Rotation um die Achse 4 bleibt somit weiterhin möglich.

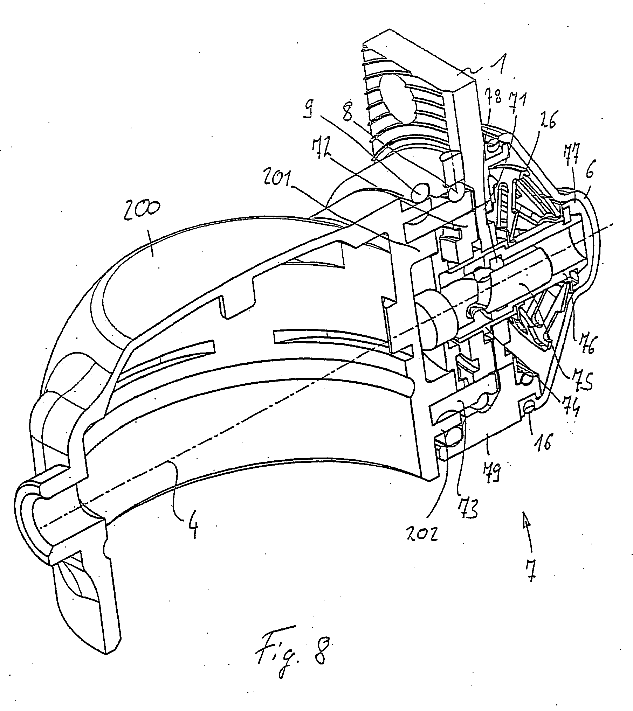

Durch erneutes Betätigen der Druckkappe 77 wird die Kugel 8 weiter in der Bahnführung des Ausrückstiftes 75 bewegt. Durch Herausbewegen der Kugel 78 aus einer sperrenden Totlage wird die Ausrückhülse 74 in die in der Figur 8 dargestellte verriegelte Position zurückkehren. Der Mechanismus funktioniert analog zu einem Kugelschreiber oder einem Push-Push-Element.

Der gleiche Mechanismus der Sperrvorrichtung 7 kann auch bei der Verriegelung und Entriegelung des Drehens um die rotatorische Achse 5 vorgenommen werden.

## Patentansprüche

1. Handprothese mit einem Chassis und Kopplungselementen zur Befestigung der Handprothese an einem Armstumpf sowie mit zumindest einem Gelenk zur Flexion und Extension der Handprothese relativ zu den Kopplungselementen, wobei das Chassis entgegen einer Federkraft in einer Neutralstellung gehalten ist, **dadurch gekennzeichnet, dass** zumindest ein Federelement (3) dem Chassis (1; 101) zugeordnet ist, das eine progressive Federkennlinie aufweist und bei zunehmendem Flexions- oder Extensionswinkel eine zunehmende Gegenkraft bereitstellt.

2. Handprothese nach Anspruch 1, **dadurch gekennzeichnet, dass** das Federelement (3) als eine Torsionsfeder ausgebildet ist.

3. Handprothese nach Anspruch 1 oder 2, **dadurch gekennzeichnet, dass** je ein Federelement (3) entgegen der Extension oder Flexion wirksam ist.

4. Handprothese nach einem der voranstehenden Ansprüche, **dadurch gekennzeichnet, dass** das Federelement (3) als Elastomerteil ausgebildet ist.

5. Handprothese nach einem der voranstehenden Ansprüche, **dadurch gekennzeichnet, dass** das Federelement (3) aus mehreren Einzelfedern besteht.

6. Handprothese nach einem der voranstehenden Ansprüche, **dadurch gekennzeichnet, dass** das Federelement (3) auswechselbar ist.

7. Handprothese nach einem der voranstehenden Ansprüche, **dadurch gekennzeichnet, dass** das Chassis (1; 101) neben der Flexion und Extension drehbar um eine Achse (5) im Wesentlichen parallel zu der Längserstreckung des Armstumpfes gelagert ist.

8. Handprothese nach einem der voranstehenden Ansprüche, **dadurch gekennzeichnet, dass** eine Sperrvorrichtung (7) in dem Gelenk (10) vorgesehen ist, die das Gelenk (10) in einer gewählten Stellung arretiert.

9. Handprothese nach Anspruch 8, **dadurch gekennzeichnet, dass** die Sperrvorrichtung (7) eine dem Gelenk (10) zugeordnete Planverzahnung (71, 72) aufweist, die zur Arretierung des Gelenkes in Eingriff bringbar ist.

10. Handprothese nach einem der voranstehenden Ansprüche, **dadurch gekennzeichnet, dass** die Achse (4) des Chassis (1; 101) des Flexions-Extensions-Gelenkes (10) im Mittelhandbereich angeordnet ist.

11. Handprothese nach einem der voranstehenden Ansprüche, **dadurch gekennzeichnet, dass** die Gelenksachse (4) in einem stumpfen Winkel zu der Längserstreckung des Armstumpfes ausgerichtet ist.

## Claims

1. Hand prosthesis with a chassis and coupling elements for fastening the hand prosthesis to an arm stump and with at least one joint for flexing and extending the hand prosthesis relative to the coupling elements, the chassis being held in a neutral position against a spring force, **characterised in that** at least one spring element (3) is associated with the chassis (1; 101), has a progressive spring characteristic and provides an increasing counterforce with an increasing flexion or extension angle.

2. Hand prosthesis according to claim 1, **characterised in that** the spring element (3) is configured as a torsion spring.

3. Hand prosthesis according to claim 1 or 2, **characterised in that** a spring element (3) is effective in each case against the extension or flexion.

4. Hand prosthesis according to any one of the preceding claims, **characterised in that** the spring element (3) is configured as an elastomer part.

5. Hand prosthesis according to any one of the preceding claims, **characterised in that** the spring element (3) consists of a plurality of individual springs.

6. Hand prosthesis according to any one of the preceding claims, **characterised in that** the spring element (3) is exchangeable.

7. Hand prosthesis according to any one of the preceding claims, **characterised in that** the chassis (1; 101), apart from the flexion and extension, is rotatably mounted about an axis (5) substantially parallel to the longitudinal extent of the arm stump.

8. Hand prosthesis according to any one of the preceding claims, **characterised in that** a locking device (7) is provided in the joint (10), which locks the joint (10) in a selected position.

9. Hand prosthesis according to claim 8, **characterised in that** the locking device (7) has a crown gearing (71, 72), which is associated with the joint (10) and can be engaged to lock the joint.

10. Hand prosthesis according to any one of the preceding claims, **characterised in that** the axis (4) of the chassis (1; 101) of the flexion-extension joint (10) is arranged in the metacarpal region.

11. Hand prosthesis according to any one of the preceding claims, **characterised in that** the joint axis (4) is aligned at an obtuse angle with respect to the longitudinal extent of the stump of the arm.

## Revendications

1. Prothèse de main avec un châssis et des éléments d'accouplement pour la fixation de la prothèse de main au moignon du bras avec au moins une articulation pour la flexion et l'extension de la prothèse de main relativement aux éléments d'accouplage, dans laquelle le châssis est maintenu de façon élastique dans une position neutre, **caractérisée en ce qu'**il est adjoint au moins un élément ressort (3) au châssis (1, 101) qui présente une courbe caractéristique progressive et fournit une force réactive qui augmente lorsque l'angle de flexion ou d'extension augmente.

2. Prothèse de main selon la revendication 1, **caractérisée en ce que** l'élément ressort (3) est agencé sous la forme d'un ressort de torsion.

3. Prothèse de main selon l'une quelconque des revendications 1 ou 2, **caractérisée en ce que** l'élément ressort (3) agit à l'encontre de l'extension ou de la flexion.

4. Prothèse de main selon l'une quelconque des revendications précédentes, **caractérisée en ce que** l'élément ressort (3) est formé d'une pièce en élastomère.

5. Prothèse de main selon l'une quelconque des revendications précédentes, **caractérisée en ce que** l'élément ressort (3) se compose de plusieurs éléments ressort individuels.

6. Prothèse de main selon l'une quelconque des revendications précédentes, **caractérisée en ce que** l'élément ressort (3) est échangeable.

7. Prothèse de main selon l'une quelconque des revendications précédentes, **caractérisée en ce que** le châssis (1, 101) en plus de la flexion et de l'extension est logé de façon mobile en rotation autour d'un axe (5) sensiblement parallèle à la direction longitudinale du moignon du bras.

8. Prothèse de main selon l'une quelconque des revendications précédentes, **caractérisée en ce qu'**un dispositif d'arrêt (7) est prévu dans l'articulation (10), qui arrête l'articulation (10) dans une position choisie.

9. Prothèse de main selon la revendication 8, **caractérisée en ce que** le dispositif d'arrêt (7) présente une denture plane (71, 72) disposée dans l'articulation (10) et qui peut entrer en prise pour l'arrêt de l'articulation (10).

10. Prothèse de main selon l'une quelconque des revendications précédentes, **caractérisée en ce que** l'axe (4) du châssis (1, 101) de l'articulation (10) de flexion-extension est disposé dans la région métacarpienne.

11. Prothèse de main selon l'une quelconque des revendications précédentes, **caractérisée en ce que** l'axe d'articulation (4) est arrangé en un angle obtus par rapport à la direction longitudinale du moignon du bras.
